# EUROPÄISCHE PATENTSCHRIFT

(11) **EP 3 064 484 B1**
(45) Veröffentlichungstag und Bekanntmachung des Hinweises auf die Patenterteilung: **20.06.2018**
(21) Anmeldenummer: 16158245.7
(22) Anmeldetag: 02.03.2016
(51) Int. Cl.: C07C 37/11, C07C 41/30, C07C 391/02, C07C 39/15, C07C 43/205

(54) **HERSTELLUNG VON 2,2'-BIARYLEN IN GEGENWART VON MOLYBDÄN(V)-CHLORID**
PREPARATION OF 2,2'-BIARYLS IN THE PRESENCE OF MOLYBDENUM (V) CHLORIDE
FABRICATION DE 2,2'-BI-ARYLE EN PRESENCE DE CHLORURE DE MOLYBDENE (V)

(30) Priorität: 05.03.2015 DE 102015203967
(43) Veröffentlichungstag der Anmeldung: 07.09.2016
(73) Patentinhaber: Evonik Degussa GmbH, 45128 Essen (DE)
(72) Erfinder: Dyballa, Katrin Marie, 45657 Recklinghausen (DE); Franke, Robert, 45772 Marl (DE); Fridag, Dirk, 45721 Haltern am See (DE); Mirion, Michael, 55122 Mainz (DE); Quell, Thomas, 55118 Mainz (DE); Waldvogel, Siegfried R., 55435 Gau-Algesheim (DE)

(56) Entgegenhaltungen:
- Siegfried R Waldvogel: "The Reaction Pattern of the MoCl 5 -Mediated Oxidative Aryl-aryl Coupling", Synlett, 1. Januar 2002 (2002-01-01), Seiten 622-623, XP55284782, Gefunden im Internet: URL:https://www.thieme-connect.com/product s/ejournals/pdf/10.1055/s-2002-22708.pdf [gefunden am 2016-06-30]
- TAPAN KANTI PAINE ET AL: "Manganese complexes of mixed O, X, O-donor ligands (X = S or Se): synthesis, characterization and catalytic reactivity", DALTON TRANSACTIONS, Nr. 15, 1. Januar 2003 (2003-01-01), Seiten 3136-3143, XP55215909, ISSN: 1477-9226, DOI: 10.1039/b304765m
- DANIELA MIRK ET AL: "Oxidative Coupling of Alkylated Anisole Derivatives Using MoCl 5", SYNLETT, Nr. 11, 1. Januar 2004 (2004-01-01), Seiten 1970-1974, XP55284779, DE ISSN: 0936-5214, DOI: 10.1055/s-2004-830870
- SIEGFRIED R. WALDVOGEL ET AL: "Oxidative transformation of aryls using molybdenum pentachloride", CHEMICAL COMMUNICATIONS - CHEMCOM, Bd. 48, Nr. 73, 1. Januar 2012 (2012-01-01), Seiten 9109-9119, XP55284791, GB ISSN: 1359-7345, DOI: 10.1039/c2cc33925k

## Beschreibung

Die Erfindung befasst sich mit der Herstellung von 2,2'-Biarylen, insbesondere von 2,2'-Biphenolen. Als Ausgangssubstanzen werden wahlweise zwei Aryle bzw. zwei Phenole oder ein 2,2'-Selenobiarylether verwendet.

Als 2,2'-Birayl bezeichnet man Verbindungen, bei denen zwei Arylgruppen über eine Einfachbindung miteinander verknüpft sind. Das einfachste Biaryl ist Biphenyl. Es ist in Schema 0 dargestellt.

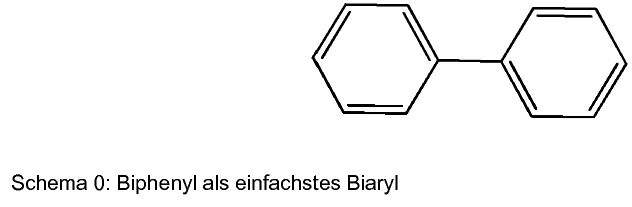

Ein 2,2'-Biphenol ist ein mit mindestens zwei OH-Gruppen subsituiertes 2,2'-Biaryl und werden auch als 2,2'-Dihydroxybiaryle bezeichnet.

Biphenole und Biaryle dienen als Synthesebausteine für katalytisch wirksame Substanzen und sind daher von industriellem Interesse. Biaryle stellen wichtige Bausteine für Flüssigkristalle, organische Vorrichtungen, Farbstoffe, Liganden für Metallkatalysatoren und finden sogar in medizinischen Bereichen Anwendungen, da diese Strukturen allgegenwärtig in biologisch aktiven, natürlich vorkommenden Produkten sind (vgl. R. Noyori, Chem. Soc. Rev. 1989, 18, 187 sowie I. Cepanec, Synthesis of Biaryls, Elsevier, New York, 2004). Insbesondere die 2,2'-Biphenole können dafür verwendetet werden; vgl. WO 2005/042547. Diese finden vor allem als Ligandkomponenten für Katalysatoren Anwendung. Dabei kann das Biphenol beispielsweise als Ligandenbaustein in der enantioselektiven Katalyse verwendet werden (vgl. Y. Chen, S. Yekta, A. K. Yudin, Chem. Rev. 2003, 103, 3155-3211; J. M. Brunel Chem. Rev. 2005, 105, 857-898; S. Kobayashi, Y. Mori, J. S. Fossey, Chem. Rev. 2011, 11, 2626-2704).

Biaryle bzw. Biphenole lassen sich durch direkte Kupplung von Arylen bzw. Phenolen zu entsprechenden Biaryl- bzw. Biphenolderivaten herstellen.

Unter den Oberbegriffen Aryle und Phenole sind in diesem Zusammenhang sowohl unsubstituierte, also auch substituierte Verbindungen zu verstehen. Die Substitution erfolgt hierbei am Benzolring.

Die Kupplung von Arylen bzw. Phenolen zu den entsprechenden Biaryl- bzw. Biphenolderivaten stellt eine große Herausforderung dar, da diese Reaktionen oftmals weder regio- noch chemoselektiv sind.

Die Synthese dieser Biphenole ist u.a. mittels elektrochemischer Verfahren möglich. Hierbei wurden Kohlenstoffelektroden wie Graphit, Glaskohlenstoff, Bor-dotierter Diamant oder Edelmetalle wie Platin verwendet; vgl. WO2010139687A1 und WO2010023258A1. Nachteiteilig bei diesen elektrochemischen Verfahren sind die zum Teil teuren Apparaturen, die extra angefertigt werden müssen. Weiterhin ist eine Hochskalierung auf den Tonnenmaßstab, wie er in der Industrie üblicherweise benötigt wird, zum Teil sehr aufwändig und in einigen Fällen sogar unmöglich.

Die direkte Kreuzkupplung ungeschützter Phenolderivate unter klassischen organischen Bedingungen ist bisher nur bei wenigen Beispielen gelungen. Hierfür wurden meist überstöchiometrische Mengen an anorganischen Oxidationsmitteln wie AlCl₃, FeCl₃, MnO₂ oder das organische 2,3-Dichlor-5,6-dicyano-1,4-benzochinon verwendet; vgl. G. Sartori, R. Maggi, F. Bigi, M. Grandi, J. Org. Chem. 1993, 58, 7271.

Alternativ werden solche Kupplungsreaktionen in einer mehrstufigen Sequenz durchgeführt. Hierbei werden Abgangsfunktionalitäten und oft giftige, komplizierte Übergangsmetallkatalysatoren, welche beispielsweise auf Palladium basieren, verwendet; vgl. L. Ackermann, Modern Arylation Methods, Wiley-VCH, Weinheim, 2009, X. Chen, K. M. Engle, D.-H. Wang, J.-Q. Yu, Angew. Chem. Int. Ed. 2009, 48, 5094-511, I. V. Seregin, V. Gevorgyan, Chem. Soc. Rev. 2007, 36, 1173-1193, G. Dyker, Handbook of C-H Transformations, Wiley-VCH, Weinheim, 2005.

Siegfried R Waldvogel, "The Reaction Pattern of the MoCl 5 -Mediated Oxidative Aryl-aryl Coupling", Synlett, 2002, No. 4, Seiten 622-624 beschreibt die oxidative Kupplung von elektronenreichen Arylen unter Zusatz von MoCl₅. Es wird der Einfluss der zugesetzten Menge an MoCl₅, bzw. die Variation des stöchiometrischen Verhältnisses von Aryl zu MoCl₅, beschrieben.

TAPAN KANTI PAINE ET AL, "Manganese complexes of mixed O, X, O-donor ligands (X = S or Se): synthesis, characterization and catalytic reactivity", DALTON TRANSACTIONS, 2003, No. 15, Seiten 3136 - 3144, beschreibt Mangan-Komplexe. Diese werden mit Biarylen gebildet, welche mit Schwefel oder Selen verbrückt sind.

Ein großer Nachteil der oben genannten Methoden zur Phenol-Kupplung ist die Notwendigkeit trockener Lösungsmittel und des Luftausschlusses. Beides bedeutet einen großen Aufwand, gerade, wenn das Verfahren großtechnisch eingesetzt werden soll.

Des Weiteren treten bei den im Stand der Technik beschriebenen Reaktionen oft toxische Nebenprodukte auf, die vom gewünschten Produkt aufwendig abgetrennt und teuer entsorgt werden müssen. Durch knapper werdende Rohstoffe (z.B. Bor und Brom) und die steigende Relevanz des Umweltschutzes steigt der Preis für solche Transformationen. Vor allem bei der Nutzung von mehrstufigen Sequenzen ist ein Wechsel von verschiedenen Lösungsmitteln notwendig, was einen hohen Aufwand darstellt und einen zusätzlichen Kostenfaktor bedeutet.

Das Problem der kostentreibenden Erforderlichkeit des Luftabschlusses wurde bereits gelöst durch die Verwendung von Selendioxid als Oxidationsmittel:
So beschreibt die zum Anmeldezeitpunkt noch nicht offen gelegte DE102014209967A1 vom 26.05.2014 die Herstellung von 2,2'-Biphenolen unter Verwendung von Selendioxid in Gegenwart einer Säure. Nachteil dieses Weges ist, dass je nach Reaktionsbedingungen auch Selenobiarylether als Nebenkomponenten entstehen. Diese müssen dann ggf. aufwendig abgetrennt werden, was das Verfahren natürlich in einer großtechnischen Umsetzung deutlich verteuert.

Die ebenfalls noch unveröffentlichte DE102014209976A1 vom selben Tage zeigt, dass anstelle der Säure ein fluoriertes Lösemittel eingesetzt werden kann, um zwei Phenole ohne Luftabschluss zu entsprechenden Biphenolen zu kuppeln. Die fluorierten Lösemittel sind aber besonders schwierig zu handhaben, insbesondere bei einem großtechnischen Einsatz, da je nach Bedingungen Flusssäure entsteht. Das ist aus sicherheitstechnischen Gründen überaus schwierig. Hinzu kommt, dass die Herstellung der fluorierten Lösemittel aufwendig und deren Einsatz entsprechend kostenintensiv ist.

Im Lichte dieses Standes der Technik lag der Erfindung die Aufgabe zu Grunde ein Verfahren zur Herstellung von 2,2'-Biarylen, insbesondere von 2,2'-Biphenolen durch Kupplung entsprechender Aryle bzw. Phenole anzugeben, welches sich industriell durchführen lässt. Dies bedeutet insbesondere, dass sich damit die gewünschten Substanzen selektiv im Tonnenmaßstab herstellen lassen, ohne dabei unbotmäßig toxische Substanzen handhaben und/oder entsorgen zu müssen. Des Weiteren sollten möglichst wenig unnütze Nebenprodukte wie etwa Selenobiarylether entstehen. Das Verfahren sollte auch Ressourcen schonend sein und deswegen möglichst ohne Brom und Bor auskommen. Auch der Einsatz von fluorierten Lösemitteln ist zu vermeiden. Schließlich ist es weiterhin wünschenswert, ohne Luftabschluss arbeiten zu können.

Gelöst wurde diese Aufgabe dadurch, die Kupplung der Aryle / Phenole weiterhin in Gegenwart von Selendioxid durchgeführt wird, jedoch zusätzlich unter der Zugabe von Molybdän-(V)-chlorid.

Es hat sich nämlich gezeigt, dass die Gegenwart des Molybdän-(V)-chlorids neben dem Selendioxid im Reaktionsgemisch die Anwesenheit eines fluorierten Lösemittels oder einer Säure überraschenderweise verzichtbar macht. Auch kann der Prozess dank des Molybdän-(V)-chlorids Brom- und Bor-frei durchgeführt werden. Dies ist beispielsweise bei klassischen palladiumkatalysierten Kupplungen nicht möglich, da dort u.a. brom- oder borhaltige Abgangsfunktionalitäten eingesetzt werden müssen. Ansonsten würde die Reaktion nicht durchführbar sein.

Das Molybdän-(V)-chlorid wirkt offenbar neben dem Selendioxid als zweites Oxidationsmittel, vielleicht darüber hinaus sogar katalytisch. Es wird in der Reaktion zwar verbraucht, nicht aber in die Biaryle bzw. Phenole eingebaut. Es wird deswegen nur in geringen Mengen benötigt. Da Molybdän-(V)-chlorid (MoCl₅) selbst biokompartibel ist und in dem Reaktionssystem auch keine toxischen Nebenprodukte bildet, überwiegen die Vorteile des zusätzlichen Einsatzes von MoCl₅.

Ein Problem beim Einsatz von Selendioxid ist, dass der entsprechende 2,2'-Selenobiarylether und das entsprechende Pummerer-Keton in großen Mengen als Nebenprodukt anfallen können. Bei ungünstiger Reaktionsführung kann es sogar vorkommen, dass der 2,2'-Selenobiarylether das Hauptprodukt der Reaktion darstellt. Dieses unerwünschte Reaktionsverhalten wird durch die Zugabe des MoCl₅ unterdrückt.

Durch Zugabe von Selendioxid als Oxidationsmittel können abhängig von den Reaktionsbedingungen 2,2'-Biphenole oder 2,2'-Selenobiarylether als Hauptprodukte der Reaktion anfallen.

In früheren Arbeiten wurde gefunden, dass sich die Reaktion durch Zugabe einer Base beziehungsweise einer Säure oder eines halogenierten Lösungsmittels in Richtung des jeweils gewünschten Produktes verschieben lässt (vgl. Anmeldungen vom 26.05.2014). Die Reaktion lässt sich durch Zugabe einer Base mit einem pKb-Wert im Bereich von 8 bis 11 gezielt in die Richtung des 2,2'-Selenobiarylether verschieben, wohingegen sich die Reaktion durch Zugabe einer Säure mit einem pKs-Wert im Bereich von 0,0 bis 5,0 oder eines halogenierten Lösungsmittels gezielt in die Richtung der 2,2'-Biphenole verschieben lässt.

Vorteile gegenüber der im Stand der Technik beschriebenen Verfahren sind, dass dabei nicht unter Feuchtigkeits- oder Sauerstoffausschluss gearbeitet werden muss. Dies stellt einen deutlichen Vorteil gegenüber anderen Syntheserouten dar. Diese direkte Methode der C,C-Kupplung ist ein effizientes und selektives Verfahren, welches sich in vorteilhafter Weise von den bestehenden mehrstufigen Syntheserouten abhebt.

Nicht umgesetzte Edukte sowie eingesetzte Lösungsmittel können destillativ zurückgewonnen und für weitere Reaktionen verwendet werden. Somit erfüllt das erfindungsgemäße Verfahren die Erfordernisse eines ökonomischen, großtechnischen Verfahrens.

Gegenstand der Erfindung ist mithin ein Verfahren zur Herstellung von 2,2'-Biarylen, bei welchem ein Reaktionsgemisch enthaltend ein erstes Aryl, ein zweites Aryl und Selendioxid erwärmt wird, wobei das erste Aryl mit dem zweiten Aryl zu dem entsprechenden 2,2'-Biaryl umgesetzt wird, wobei das Reaktionsgemisch zusätzlich Molybdän(V)-chlorid enthält.

In welcher Reihenfolge die beiden Aryle, das Selendioxid und das MoCl5 zu dem Reaktionsgemisch hinzuzugefügt werden, ist unerheblich. Grundsätzlich können auch weitere Komponenten in dem Reaktionsgemisch wie beispielsweise Lösungsmittel, Säure oder Base enthalten sein.

Vorzugsweise handelt es sich bei dem bei dem ersten Aryl und/oder bei dem zweiten Aryl um eine Verbindung gemäß der allgemeinen Formel **I:** 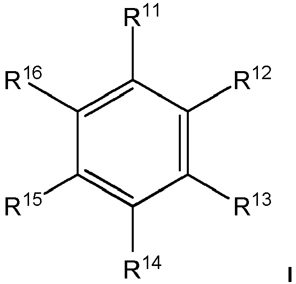 wobei R¹¹, R¹², R¹³, R¹⁴, R¹⁵, R¹⁶ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl, -Cl, -F, -I, -OC=O-(C₁-C₁₂)-Alkyl, -NH₂, -N[(C₁-C₁₂)-Alkyl]₂;
wobei zwei benachbarte Reste können zu einem kondensierten System miteinander verbunden sein können aber nicht müssen,
und wobei die genannten Alkyl- und Arylgruppen substituiert sein können aber nicht müssen,
und mindestens ein Rest R¹¹, R¹², R¹³, R¹⁴, R¹⁵ oder R¹⁶ gleich -H ist.

Weiter vorzugsweise sind R¹¹, R¹², R¹³, R¹⁴, R¹⁵, R¹⁶ jeweils unabhängig voneinander ausgewählt aus:
-H, -(C₁-C₁₂)-Alkyl, -O-(C₁-C₁₂)-Alkyl, -(C₆-C₂₀)-Aryl, -O-(C₆-C₂₀)-Aryl, -Cl, -F, -I,
wobei die genannten Alkyl- und Arylgruppen substituiert sein können aber nicht müssen,
und mindestens ein Rest R¹¹, R¹², R¹³, R¹⁴, R¹⁵ oder R¹⁶ gleich -H ist.

In einer speziellen Ausführungsform des Verfahrens ist mindestens ein
Rest R¹¹, R¹², R¹³, R¹⁴, R¹⁵ oder R¹⁶ :
-O-(C₁-C₁₂)-Alkyl,

In einer noch spezielleren Ausführungsform des Verfahrens sind R¹¹, R¹³, R¹⁴ ausgewählt aus: -O-(C₁-C₁₂)-Alkyl.

In der speziellsten Ausführungsform des Verfahrens sind R¹¹, R¹³, R¹⁴ ausgewählt aus: -O-CH₃.

In einer Variante des Verfahrens entspricht das erste Aryl dem zweiten Aryl, die beiden Aryle sind also identisch. Bei dieser Variante handelt es sich also um eine Homokupplung zweier gleicher Aryle. Durch die ortho-ortho-Kupplung entstehen so die gewünschten 2,2'-Biarylen.

In dem vorliegenden Fall wird das 2,2'-Biphenol als Hauptprodukt angestrebt, da Biphenole wertvolle Bausteine zur Synthese von Liganden für metallorganische Komplexkatalysatoren sind und andere Anwendungen sind.

Eine besonders bevorzugte Ausführungsform der Erfindung dient demnach der Herstellung von 2,2'-Biphenolen. Diese ist folglich dadurch gekennzeichnet, dass es sich bei dem ersten Aryl um ein erstes Phenol handelt, dass es sich bei dem zweiten Aryl um ein zweites Phenol handelt, und dass das erste Phenol mit dem zweiten Phenol zu dem entsprechenden 2,2'-Biphenol umgesetzt wird.

Bei dem ersten Phenol und/oder bei dem zweiten Phenol handelt es sich vorzugsweise um eine Verbindung gemäß der allgemeinen Formel **II**: 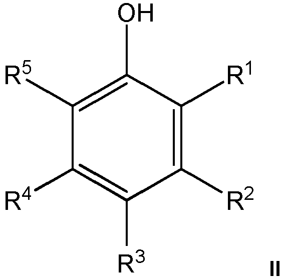 wobei R¹, R², R³, R⁴, R⁵ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl, -Cl, -F, -I, -OC=O-(C₁-C₁₂)-Alkyl,
wobei zwei benachbarte Reste zu einem kondensierten System miteinander verbunden sein können aber nicht müssen,
wobei die genannten Alkyl- und Arylgruppen substituiert sein können aber nicht müssen,
und wobei mindestens R¹ oder R⁵ gleich -H ist.

Vorzugsweise sollten R¹, R², R³, R⁴, R⁵ jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Cl, -F, -I,
wobei die genannten Alkyl- und Arylgruppen substituiert sein können, aber nicht müssen,
und wobei mindestens R¹ oder R⁵ gleich -H ist.

(C₁-C₁₂)-Alkyl und O-(C₁-C₁₂)-Alkyl können jeweils unsubstituiert oder durch einen oder mehrere gleiche oder verschiedene Reste substituiert sein, die ausgewählt sind aus:
(C₃-C₁₂)-Cycloalkyl, (C₃-C₁₂)-Heterocycloalkyl, (C₆-C₂₀)-Aryl, Fluor, Chlor, Cyano, Formyl, Acyl oder Alkoxycarbonyl.

(C₆-C₂₀)-Aryl und O-(C₆-C₂₀)-Aryl können jeweils unsubstituiert oder durch einen oder mehrere gleiche oder verschiedene Reste substituiert sein, die ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I),-COO-(C₁-C₁₂)-Alkyl, -CONH-(C₁-C₁₂)-Alkyl, -(C₆-C₂₀)-Aryl-CON[(C₁-Cl₂)-Alkyl]₂, -CO-(C₁-C₁₂)-Alkyl, -CO-(C₆-C₂₀)-Aryl, -COOH, -OH, -SO₃H; -SO₃Na, -NO₂, -CN, -NH₂, -N[(C₁-C₁₂)-Alkyl]₂.

Im Rahmen der Erfindung umfasst der Ausdruck (C₁-C₁₂)-Alkyl geradkettige und verzweigte Alkylgruppen. Vorzugsweise handelt es sich dabei um unsubstituierte geradkettige oder verzweigte (C₁-C₈)-Alkylgruppen und ganz bevorzugt (C₁-C₆)-Alkylgruppen. Beispiele für (C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Dimethylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Die Erläuterungen zum Ausdruck -(C₁-C₁₂)-Alkyl gelten auch für die Alkylgruppen in -O-(C₁-C₁₂)-Alkyl, also in -(C₁-C₁₂)-Alkoxy. Vorzugsweise handelt es sich dabei um unsubstituierte geradkettige oder verzweigte -(C₁-C₆)-Alkoxygruppen.

Substituierte (C₁-C₁₂)-Alkylgruppen und substituierte (C₁-C₁₂)-Alkoxygruppen können in Abhängigkeit von ihrer Kettenlänge, einen oder mehrere Substituenten aufweisen. Die Substituenten sind vorzugsweise unabhängig voneinander ausgewählt sind aus:
-(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl,

In einer Variante des Verfahrens entspricht das erste Phenol dem zweiten Phenol. Das erste Phenol und das zweite Phenol sind mithin identisch Bei dieser Variante handelt es sich also um eine Homokupplung zweier gleicher Phenole. Durch die ortho-ortho-Kupplung entstehen so die gewünschten 2,2'-Biphenole.

Im Rahmen der vorliegenden Arbeiten wurde zusätzlich gefunden, dass sich 2,2'-Biaryle nicht nur durch Kopplung der Aryle, sondern auch durch Umsetzung von 2,2'-Selenobiarylethern bilden lassen, allerdings nur in Gegenwart von MoCl₅. Da die Anwesenheit des Molybdän-(V)-Chlorids auch hier erfolgswesentlich ist, und dieselben Biaryle entstehen, liegt insoweit ein gemeinsames erfinderisches Konzept zu Grunde, nämlich die Herstellung von 2,2'-Biarylen in Gegenwart von Molybdän(V)-chlorid.

Ein weiterer Gegenstand der Erfindung ist mithin ein Verfahren zur Herstellung von 2,2'-Biarylen, bei welchem ein 2,2'-Selenobiarylether in Gegenwart von Molybdän(V)-chlorid erwärmt und zu einem 2,2'-Biaryl umgesetzt wird.

Die erforderlichen 2,2'-Selenobiarylether waren bereits im Rahmen früherer Arbeiten gefunden worden; vgl. DE102014209974A1, ebenfalls am 26.05.2014 eingereicht und daher zum Anmeldezeitpunkt noch unveröffentlicht.

Bisher wurden die 2,2'-Selenobiarylether als unnütze Nebenprodukte angesehen, die aufwendig abgetrennt und entsorgt werden mussten. Dank des nun gefundenen Reaktionsweg lassen sich diese 2,2'-Selenobiarylether fortan als Edukte für die gewünschten 2,2'-Biaryle verwenden, sodass sie kein unerwünschtes Nebenprodukt mehr darstellen, sondern vielmehr ein weiteres Wertprodukt.

Durch die Zugabe des MoCl₅ zu 2,2'-Selenobiarylethern lassen sich als die korrespondierenden 2,2'-Biaryle bilden.

Es wurde gefunden, dass sich ausgehend vom 2,2'- Selenobiarylether die Reaktion durch Zugabe des MoCl₅ gezielt in die Richtung des 2,2'-Biaryls oder 2,2'-Biphenols verschieben lässt. Dies ist besonders vorteilhaft, da diese Verbindungen im Vergleich zu den korrespondierenden 2,2'- Selenobiarylethern die interessanteren Produkte mit einer Vielzahl von großtechnischen Anwendungsgebieten aufweist.

Bei dem 2,2'-Selenobiarylether handelt es sich vorzugsweise um eine Verbindung gemäß der allgemeinen Formel **III**: 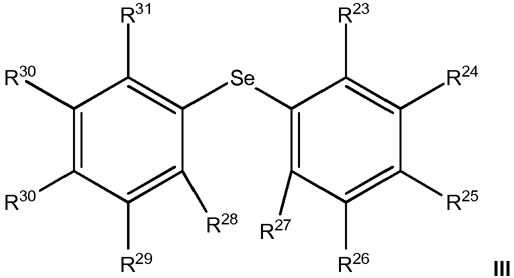 wobei R²³, R²⁴, R²⁵, R²⁶, R²⁷, R²⁸, R²⁹, R³⁰, R³¹ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl, - Cl, -F, -I,
   -OC=O-(C₁-C₁₂)-Alkyl, -NH₂, -N[(C₁-C₁₂)-Alkyl]₂; OH;
wobei zwei benachbarte Reste zu einem kondensierten System miteinander verbunden sein können aber nicht müssen,
und wobei die genannten Alkyl- und Arylgruppen substituiert sein können aber nicht müssen.

Weiter vorzugsweise sollten R²³, R²⁴, R²⁵, R²⁶, R²⁷, R²⁸, R²⁹, R³⁰, R³¹ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl, -Cl, -F, -I, -OH.

Ganz besonders bevorzugt sind R²³, R²⁴, R²⁵, R²⁶, R²⁷, R²⁸, R²⁹, R³⁰, R³¹ jeweils unabhängig voneinander ausgewählt sind aus: -H, -(C₁-C₁₂)-Alkyl, -O-(C₁-C₁₂)-Alkyl.

In einer Variante des Verfahrens sind R²³, R²⁴, R²⁵ , R²⁶, R²⁷, R²⁸, R²⁹, R³⁰, R³¹ jeweils unabhängig voneinander ausgewählt sind aus:
-H, -(C₁-C₁₂)-Alkyl, -O-(C₁-C₁₂)-Alkyl, OH;

In einer speziellen Variante des Verfahrens sind R²³, R²⁴, R²⁵, R²⁶, R²⁷, R²⁸, R²⁹, R³⁰, R³¹ jeweils unabhängig voneinander ausgewählt sind aus:
-H, -(C₁-C₁₂)-Alkyl, -O-(C₁-C₁₂)-Alkyl,

In einer noch spezielleren Ausführungsform des Verfahrens sind R²³, R³¹ ausgewählt aus: -OH.

In der speziellsten Ausführungsform sind R²³, R³¹ -OCH₃.

Die Reaktion des Selenobiarylether zu dem Biaryl erfolgt bei Temperatur in dem Bereich von 50 °C bis 110 °C. Hierbei ist der Bereich von 60 °C bis 100 °C bevorzugt, und der Bereich von 70 °C bis 90 °C besonders bevorzugt.

Unabhängig davon, ob die Biaryle bzw. Biphenole aus zwei Arylen/Phenolen oder aus dem 2,2'-Selenobiarylether hergestellt wird, sollte die Reaktion unter den folgenden Randbedingungen durchgeführt werden:
Auf die Anwesenheit eines fluorierten Lösemittels und/oder einer Säure sollte tunlichst verzichtet werden. Beides ist im Labormaßstab zwar möglich, im industriellen Maßstab jedoch nicht praktikabel.

Gleichwohl kann die Reaktion durchgeführt werden in Anwesenheit einer Base um die Reaktion in Richtung der gewünschten Produkte zu begünstigen. Die verwendete Base sollte einen pKb-Wert im Bereich von 8 bis 11 haben. Bei der Base könnte es sich um Pyridin, um Chinolin, um eine Aminbase wie beispielsweise Triethylamin oder um Dimethylformamid handeln. Besonders bevorzugt ist Pyridin.

Neben der Base kann das Reaktionsgemisch auch ein Lösemittel umfassen, wie beispielsweise Tetrahydrofuran, Ethylenglycoldimethylether, Bis(2-methoxyethyl)ether, Diethylether, Toluol. Alle diese Lösemittel sind frei von Fluor und daher gut verfügbar. Der Zweck des Lösemittels besteht darin, eine gute Vermischung und Rührbarkeit der verschiedenen Komponenten miteinander zu gewährleisten.

Ganz besonders bevorzugt wird die Base als Lösemittel verwendet. Die Base ist also gleichzeitig Lösungsmittel; Base und Lösemittel die identische Substanz. Dies erlaubt Pyridin, weswegen die Reaktion auch bevorzugt in Anwesenheit von Pyridin durchgeführt wird.

Ein besonderer Vorteil der hier beschriebenen Reaktionssysteme ist, dass diese nicht anfällig sind gegen feuchte Umgebungsluft, also einem Gemisch aus Wasserdampf, Sauerstoff und Stickstoff, ggf. sogar zusätzlich auch flüssiges Wasser. Deswegen braucht nicht unter Luftabschluss gearbeitet zu werden, was die Durchführung der Reaktion erheblich vereinfacht und erst industriell praktikabel macht. Die Möglichkeit, das Verfahren in Anwesenheit feuchter Luft durchzuführen, ist daher von besonderem Interesse.

Vorzugsweise wird das Selendioxid in einem molaren Verhältnis bezogen auf die Summe des ersten und des zweiten Aryls zugegeben, welches in einem Bereich von 0,25 bis 1,2 liegt. Hierbei ist der Bereich von 0,25 bis 0,9 bevorzugt, und der Bereich von 0,4 bis 0,7 besonders bevorzugt.

Dass das Selendioxid in einer unterstöchiometrischen Menge eingesetzt werden kann, stellt einen weiteren Vorteil gegenüber der im Stand der Technik beschriebenen Reaktion mit anderen anorganischen Oxidationsmitteln wie beispielsweise AlCl₃, FeCl₃ oder MnO₂ dar. Grundsätzlich sollte Selendioxid nicht in großem Mengen konsumiert werden, weswegen sein unterstöchiometrischer Einsatz bevorzugt ist.

Hinsichtlich der Menge des zuzusetzenden Molybdän-(V)-Chlorids lassen sich folgende Angaben machen: 0,01 bis 0,6 Äquivalente, bevorzugt 0,05 bis 0,2 Äquivalente bezogen auf das Edukt.

Vorzugsweise erfolgt die Reaktion über einen Zeitraum im Bereich von 5 Minuten bis 24 Stunden. Hierbei ist der Bereich von 15 Minuten bis 12 Stunden bevorzugt, und der Bereich von 15 Minuten bis 6 Stunden besonders bevorzugt.

Im Folgenden wird die Erfindung anhand von Ausführungsbeispielen näher erläutert.

### Analytik

### NMR-Spektroskopie

Die NMR-spektroskopischen Untersuchungen wurden an Multikernresonanzspektrometern des Typs AC 300 oder AV II 400 der Firma *Bruker*, Analytische Messtechnik, Karlsruhe, durchgeführt. Als Lösungsmittel wurde CDCl₃ verwendet. Die ¹H- und ¹³C-Spektren wurden gemäß dem Restgehalt an nicht deuteriertem Lösungsmittel nach der *NMR Solvent Data Chart* der Fa. *Cambridge Isotopes Laboratories*, USA, kalibriert. Die Zuordnung der ¹H- und ¹³C-Signale erfolgte teilweise mit Hilfe von H,H-COSY, H,H-NOESY, H,C-HSQC und H,C-HMBC-Spektren. Die chemischen Verschiebungen sind als δ-Werte in ppm angegeben. Für die Multiplizitäten der NMR-Signale wurden folgende Abkürzungen verwendet: s (Singulett), bs (breites Singulett), d (Dublett), t (Triplett), q (Quartett), m (Multiplett), dd (Dublett von Dublett), dt (Dublett von Triplett), tq (Triplett von Quartett). Alle Kopplungskonstanten J wurden mit der Anzahl der eingeschlossenen Bindungen in Hertz (Hz) angegeben. Die bei der Signalzuordnung angegebene Nummerierung entspricht der in den Formelschemata angegebenen Bezifferung, die nicht mit der IUPAC-Nomenklatur übereinstimmen muss.

### Allgemeine Arbeitsvorschrift (AAV1)

Das jeweilige Phenol oder Methoxyphenol wurde im entsprechenden Lösungsmittel gelöst (8.2 m). Das Reaktionsgemisch wurde auf 60 oder 85 °C erhitzt und Selendioxid (0.6 Äquivalente) wurde unter Rühren zugegeben. Das Lösungsmittel wurde im Vakuum destilliert (Temperatur <70° C). Eine Fritte wurde zum Filtrieren der festen Bestandteile genutzt und dafür mit 2,5 cm (unten) Kieselgel und 2,5 cm Zeolith (oben) befüllt. Der Destillationsrückstand wurde im Laufmittel aufgenommen und auf die Filtrationssäule geben. Mit Cyclohexan:Essigester (95:5) wurde das Produkt von der Fritte gewaschen in Fraktionen gesammelt und destillativ vom Laufmittel befreit

### Synthese der Selenobiarylether

### Bis(3,5-dimethyl-2-hydroxyphenyl)selen (1)

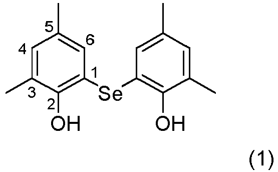

Die Durchführung der Reaktion erfolgte gemäß AAV1 in einem verschraubbaren Reagenzglas. Hierzu wurde 2,4-Dimethylphenol (1.00 g, 8.2 mmol) und Selendioxid (0.54 g, 4.9 mmol) in Pyridin (1 mL, 8.2 m) gelöst und 5h auf 60 °C erhitzt. Das Produkt **1** (0.50 g, 3.1 mmol, 38%) wurde als farbloser kristalliner Feststoff erhalten.

GC (hart, HP-5): t_{R}= 14.2 min. ¹H-NMR (400 MHz, CDCl₃): δ = 7.12 (s, 2H, 6-H), 6.91 (s, 2H, 4-H), 5.97 (s,2H, OH), 2.23 (s, 6H, 3-CH₃) 2.23 (s, 6H, 5-CH₃) ppm. ¹³C-NMR (100 MHz, CDCl₃): 151.7 (C-2), 133.2 (C-3), 133.1 (C-5), 130.4 (C-4), 124.2 (C-6), 114.9 (C-1), 20.3 (5-CH₃), 16.5 (3-CH₃) ppm. ⁷⁷Se-NMR (76 MHz, CDCl₃): δ = 163.36 ppm. MS (ESI⁺): *mlz* = 345,04 [M+Na]⁺, 667,08 [2M+Na]⁺.

### Bis(2,3,5-trimethoxyphenyl)selen (2)

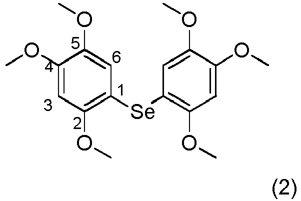

### 3,3',5,5'-Tetramethylbiphenyl-2,2'-diol (3)

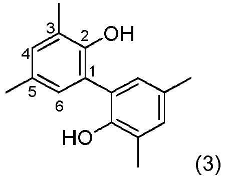

### Synthese ausgehend vom Phenol

Die Durchführung der Reaktion erfolgte gemäß AAV1 in einem verschraubbaren Reagenzglas. Hierzu wurde 2,4-Dimethylphenol (1.00 g, 8.2 mmol) und Selendioxid (0.54 g, 4.9 mmol) in Ameisensäure (1 mL, 8.2 m) gelöst und 2h auf 70 °C erhitzt. Das Produkt **3** (0.61 g, 5.0 mmol, 61 %) wurde als beiger kristalliner Feststoff erhalten.

Referenz im Laborjournal (MMI-): 394, 395, 397, 400, 427, 443, 474.

### Synthese ausgehend von Selenobiarylether (1)(erfindungsgemäß)

Der Selenobiarylether **1** (500 mg, 1.56 mmol) wurde in Dichlormethan (10 mL) gelöst. Bei 22 °C wurde Molybdänpentachlorid in Dichlormethan (935 mg in 2 mL, 3.42 mmol) zugegeben. Zum Beenden der Reaktion wurde nach 20 min Wasser (10 mL) zugegeben. Die organische Phase wurde abgetrennt und das Lösungsmittel wurde im Vakuum destilliert. Der Destillationsrückstand wurde im Laufmittel aufgenommen und auf die Filtrationssäule geben. Mit Cyclohexan:Essigester (95:5) wurde das Produkt von der Filtrationssäule gewaschen und destillativ vom Laufmittel befreit. Das Produkt **3** konnte in kleinen Mengen erhalten werden.

GC (hart, HP-5): t_{R}= 12.3 min. R_{f}= 0.2 (CH:EE 98:2). ¹H-NMR (300 MHz, CDCl₃): δ = 7.00 (s,2H, 6-H), 6.87 (s, 2H, 4-H), 5.07 (s,2H, OH), 2.27 (s, 12H, 3-CH₃, 5-CH₃) ppm. ¹³C-NMR (75 MHz, CDCl₃): δ = 149.2 (C-2),132.1 (C-4), 130.0 (C-5), 128.5 (C-6), 125.1 (C-3), 122.1 (C-1), 20.4 (5-CH₃), 16.2 (3-CH₃) ppm. HRMS (ESI+) *m*/*z* für C₂₉H₃₄O₄Na [M + Na]⁺ berechnet: 469.2355, gefunden 469.2352.

### 1,2,4,1',2',4'-Hexamethoxy-5,5'-biphenyl (4) (erfindungsgemäß)

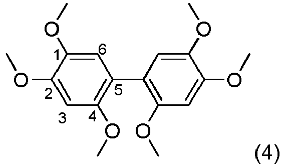

Der Selenobiarylether **2** (500 mg, 1.21 mmol) wurde in Dichlormethan (10 mL) gelöst. Bei 22 °C wurde Molybdänpentachlorid in Dichlormethan (727 mg in 1.5 mL, 2.66 mmol) zugegeben. Zum Beenden der Reaktion wurde nach 20 min Wasser (10 mL) zugegeben. Die organische Phase wurde abgetrennt und das Lösungsmittel wurde im Vakuum destilliert. Der Destillationsrückstand wurde im Laufmittel aufgenommen und auf eine Filtrationssäule geben. Mit Cyclohexan:Essigester (95:5) wurde das Produkt von der Filtrationssäule gewaschen und in Fraktionen gesammelt die destillativ vom Laufmittel befreit wurden. Das Produkt **4** (399 mg, 1.19 mmol, 99%) wurde als beiger kristalliner Feststoff erhalten.

GC (hart, HP-5): t_{R}= 15.3 min. ¹H-NMR (400 MHz, CDCl₃): δ = 6.82 (s, 2H), 6.63 (s, 2H), 3.93 (s, 6H), 3.84 (s, 6H), 3.76 (s, 6H) ppm. ¹³C-NMR (101 MHz, CDCl₃): δ = 151.38, 148.93, 143.02, 119.05, 115.45, 98.50, 57.04, 56.68, 56.25 ppm.

Die Ergebnisse zeigen, dass das erfindungsgemäße Verfahren eine Syntheseroute darstellt, mit welcher 2,2'-Biaryle selektiv, in guter Ausbeute ausgehend von den 2,2'-Selenobiarylethern hergestellt werden können.

Beachtlich ist, dass die verwendeten Substanzen im Vorfeld nicht getrocknet wurden und die Arbeiten auch nicht unter inerten Bedingungen durchgeführt wurden. Normalerweise würde man - wenn man unter Luftausschluss arbeitet - alle Lösemittel und Feststoffe vorher trocknen und in so genannten Schlenkkolben arbeiten. Schlenkkolben sind Reaktionsgefäße, die erst evakuiert und dann mit Argon oder Stickstoff gefüllt werden bis die Luft verdrängt ist, sodass man letztendlich nur unter Schutzgas (Argon/Stickstoff) arbeitet. Dies wurde in den vorliegenden Versuchen nicht gemacht. Folglich wurden alle Versuche in Gegenwart feuchter Luft, also der Umgebungsluft des Labors durchgeführt. Es steht somit zu erwarten, dass auch bei der erfindungsgemäßen Produktion der Biaryle / Biphenole im Tonnenmaßstab kein Luftabschluss notwendig sein wird, wodurch eine erhebliche Verfahrensvereinfachung und Kostenreduktion erzielt werden wird.

## Patentansprüche

1. Verfahren zur Herstellung von 2,2'-Biarylen, bei welchem ein Reaktionsgemisch enthaltend ein erstes Aryl, ein zweites Aryl und Selendioxid erwärmt wird, wobei das erste Aryl mit dem zweiten Aryl zu dem entsprechenden 2,2'-Biaryl umgesetzt wird, **dadurch gekennzeichnet, dass** das Reaktionsgemisch zusätzlich Molybdän(V)-chlorid enthält.

2. Verfahren nach Anspruch 1, **dadurch gekennzeichnet, dass** es sich bei dem ersten Aryl und/oder bei dem zweiten Aryl um eine Verbindung gemäß der allgemeinen Formel **I** handelt: 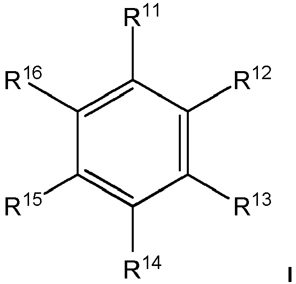 wobei R¹¹, R¹², R¹³, R¹⁴, R¹⁵, R¹⁶ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl, -Cl, -F, -I, -OC=O-(C₁-C₁₂)-Alkyl, -NH₂, -N[(C₁-C₁₂)-Alkyl]₂;
wobei zwei benachbarte Reste können zu einem kondensierten System miteinander verbunden sein können aber nicht müssen,
und wobei die genannten Alkyl- und Arylgruppen substituiert sein können aber nicht müssen, und mindestens ein Rest R¹¹, R¹², R¹³, R¹⁴, R¹⁵ oder R¹⁶ gleich -H ist.

3. Verfahren nach Anspruch 2, **dadurch gekennzeichnet, dass** R¹¹, R¹², R¹³, R¹⁴, R¹⁵, R¹⁶ jeweils unabhängig voneinander ausgewählt aus:
-H, -(C₁-C₁₂)-Alkyl, -O-(C₁-C₁₂)-Alkyl, -(C₆-C₂₀)-Aryl, -O-(C₆-C₂₀)-Aryl, -Cl, -F, -I,
wobei die genannten Alkyl- und Arylgruppen substituiert sein können aber nicht müssen, und mindestens ein Rest R¹¹, R¹², R¹³, R¹⁴, R¹⁵ oder R¹⁶ gleich -H ist.

4. Verfahren nach Anspruch 2 oder 3, **dadurch gekennzeichnet, dass** das erste Aryl und das zweite Aryl identisch sind.

5. Verfahren nach Anspruch 1 zur Herstellung von 2,2'-Biphenolen, **dadurch gekennzeichnet, dass** es sich bei dem ersten Aryl um ein erstes Phenol handelt, dass es sich bei dem zweiten Aryl um ein zweites Phenol handelt, und dass das erste Phenol mit dem zweiten Phenol zu dem entsprechenden 2,2'-Biphenol umgesetzt wird.

6. Verfahren nach Anspruch 5, **dadurch gekennzeichnet, dass** es sich bei dem ersten Phenol und/oder bei dem zweiten Phenol um eine Verbindung gemäß der allgemeinen Formel **II** handelt: 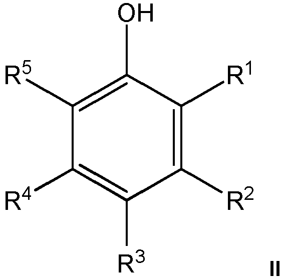 wobei R¹, R², R³, R⁴, R⁵ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl, -Cl, -F, -I, -OC=O-(C₁-C₁₂)-Alkyl,
wobei zwei benachbarte Reste zu einem kondensierten System miteinander verbunden sein können aber nicht müssen,
wobei die genannten Alkyl- und Arylgruppen substituiert sein können aber nicht müssen, und wobei mindestens R¹ oder R⁵ gleich -H ist.

7. Verfahrens nach den Anspruch 6, wobei
R¹, R², R³, R⁴, R⁵ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl, -Cl, -F, -I,
wobei die genannten Alkyl- und Arylgruppen substituiert sein können, aber nicht müssen, und wobei mindestens R¹ oder R⁵ gleich -H ist.

8. Verfahren nach Anspruch 6 oder 7, **dadurch gekennzeichnet, dass** das erste Phenol und das zweite Phenol identisch sind.

9. Verfahren zur Herstellung von 2,2'-Biarylen, **dadurch gekennzeichnet, dass** ein 2,2'-Selenobiarylether in Gegenwart von Molybdän(V)-chlorid erwärmt und zu einem 2,2'-Biaryl umgesetzt wird.

10. Verfahren nach Anspruch 11,
wobei es sich bei dem 2,2'-Selenobiarylether um eine Verbindung gemäß der allgemeinen Formel **III** handelt: 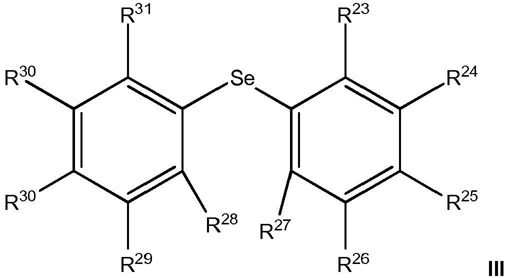 wobei R²³, R²⁴, R²⁵, R²⁶, R²⁷, R²⁸, R²⁹, R³⁰, R³¹ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl, - Cl, -F, -I, -OC=O-(C₁-C₁₂)-Alkyl, -NH₂, -N[(C₁-C₁₂)-Alkyl]₂; OH;
wobei zwei benachbarte Reste zu einem kondensierten System miteinander verbunden sein können aber nicht müssen,
und wobei die genannten Alkyl- und Arylgruppen substituiert sein können aber nicht müssen.

11. Verfahren nach Anspruch 10,
wobei R²³, R²⁴, R²⁵, R²⁶, R²⁷, R²⁸, R²⁹, R³⁰, R³¹ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl, -Cl, -F, -I, -OH.

12. Verfahren nach Anspruch 10 oder 11,
wobei R²³, R²⁴, R²⁵, R²⁶, R²⁷, R²⁸, R²⁹, R³⁰, R³¹ jeweils unabhängig voneinander ausgewählt sind aus: -H, -(C₁-C₁₂)-Alkyl, -O-(C₁-C₁₂)-Alkyl.

13. Verfahren nach Anspruch 1 oder einem der Ansprüche 2 bis 13, durchgeführt in Abwesenheit eines fluorierten Lösemittels und/oder in Abwesenheit einer Säure.

14. Verfahren nach Anspruch 1 oder einem der Ansprüche 2 bis 13 durchgeführt in Anwesenheit einer Base ausgewählt aus Pyridin, Chinolin, Aminbasen, Dimethylformamid und/oder in Anwesenheit eines Lösemittels ausgewählt aus Tetrahydrofuran, Ethylenglycoldimethylether, Bis(2-methoxyethyl)ether, Diethylether, Toluol.

15. Verfahren nach Anspruch 1 oder einem der Ansprüche 2 bis 14, durchgeführt in Anwesenheit feuchter Luft.

## Claims

1. Process for preparing 2,2'-biaryls, in which a reaction mixture comprising a first aryl, a second aryl and selenium dioxide is heated, the first aryl being reacted with the second aryl to give the corresponding 2,2'-biaryl, **characterized in that** the reaction mixture additionally comprises molybdenum(V) chloride.

2. Process according to Claim 1, **characterized in that** the first aryl and/or the second aryl is a compound of the general formula **I:** 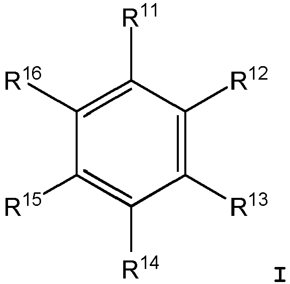 where R¹¹, R¹², R¹³, R¹⁴, R¹⁵, R¹⁶ are each independently selected from:
-H, -(C₁-C₁₂)-alkyl, -O-(C₁-C₁₂)-alkyl, -(C₆-C₂₀)-aryl, -O-(C₆-C₂₀)-aryl, -Cl, -F, -I, -OC=O-(C₁-C₁₂) -alkyl, -NH₂,-N[(C₁-C₁₂)-alkyl]₂;
where two adjacent radicals may may but need not be joined to one another to give a fused system,
and where the alkyl and aryl groups mentioned may but need not be substituted,
and at least one R¹¹, R¹², R¹³, R¹⁴, R¹⁵ or R¹⁶ radical is - H.

3. Process according to Claim 2, **characterized in that** R¹¹, R¹², R¹³, R¹⁴, R¹⁵, R¹⁶ each independently selected from:
-H, -(C₁-C₁₂)-alkyl, -O-(C₁-C₁₂)-alkyl, -(C₆-C₂₀)-aryl, -O-(C₆-C₂₀)-aryl, -Cl, -F, -I,
where the alkyl and aryl groups mentioned may but need not be substituted,
and at least one R¹¹, R¹², R¹³, R¹⁴, R¹⁵ or R¹⁶ radical is - H.

4. Process according to Claim 2 or 3, **characterized in that** the first aryl and the second aryl are identical.

5. Process according to Claim 1 for preparing 2,2'-biphenols, **characterized in that** the first aryl is a first phenol, **in that** the second aryl is a second phenol, and **in that** the first phenol is reacted with the second phenol to give the corresponding 2,2'-biphenol.

6. Process according to Claim 5, **characterized in that** the first phenol and/or the second phenol is a compound of the general formula **II:** 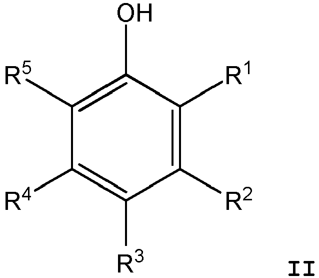 wherein R¹, R², R³, R⁴, R⁵ are each independently selected from:
-H, -(C₁-C₁₂)-alkyl, -O-(C₁-C₁₂)-alkyl, -(C₆-C₂₀)-aryl, -O-(C₆-C₂₀)-aryl, -Cl, -F, -I,
-OC=O-(C₁-C₁₂)-alkyl,
where two adjacent radicals may but need not be joined to one another to give a fused system,
where the alkyl and aryl groups mentioned may but need not be substituted,
and where at least R¹ or R⁵ is -H.

7. Process according to Claim 6, where R¹, R², R³, R⁴, R⁵ are each independently selected from:
-H, -(C₁-C₁₂)-alkyl, -O-(C₁-C₁₂)-alkyl, -(C₆-C₂₀)-aryl, -O-(C₆-C₂₀)-aryl, -Cl, -F, -I,
where the alkyl and aryl groups mentioned may but need not be substituted,
and where at least R¹ or R⁵ is -H.

8. Process according to Claim 6 or 7, **characterized in that** the first phenol and the second phenol are identical.

9. Process for preparing 2,2'-biaryls, **characterized in that** a 2,2'-selenobiaryl ether is heated in the presence of molybdenum(V) chloride and converted to a 2,2'-biaryl.

10. Process according to Claim 11,
wherein the 2,2'-selenobiaryl ether is a compound of the general formula **III:** 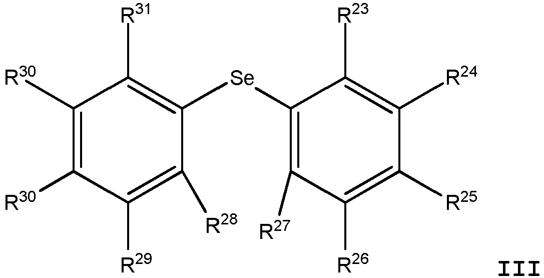 where R²³, R²⁴, R²⁵, R²⁶, R²⁷, R²⁸, R²⁹, R³⁰, R³¹ are each independently selected from:
-H, -(C₁-C₁₂)-alkyl, -O-(C₁-C₁₂)-alkyl, -(C₆-C₂₀)-aryl, -O-(C₆-C₂₀)-aryl, -Cl, -F, -I,
-OC=O-(C₁-C₁₂)-alkyl, -NH₂, -N[(C₁-C₁₂)-alkyl]₂; OH;
where two adjacent radicals may but need not be joined to one another to give a fused system,
and where the alkyl and aryl groups mentioned may but need not be substituted.

11. Process according to Claim 10,
where R²³, R²⁴, R²⁵, R²⁶, R²⁷, R²⁸, R²⁹, R³⁰, R³¹ are each independently selected from:
-H, -(C₁-C₁₂)-alkyl, -O-(C₁-C₁₂)-alkyl, -(C₆-C₂₀)-aryl, -O-(C₆-C₂₀)-aryl, -Cl, -F, -I, -OH.

12. Process according to Claim 10 or 11,
where R²³, R²⁴, R²⁵, R²⁶, R²⁷, R²⁸, R²⁹, R³⁰, R³¹ are each independently selected from: -H, -(C₁-C₁₂)-alkyl, -O-(C₁-C₁₂)-alkyl.

13. Process according to Claim 1 or any of Claims 2 to 13, conducted in the absence of a fluorinated solvent and/or in the absence of an acid.

14. Process according to Claim 1 or any of Claims 2 to 13, conducted in the presence of a base selected from pyridine, quinoline, amine bases, dimethylformamide, and/or in the presence of a solvent selected from tetrahydrofuran, ethylene glycol dimethyl ether, bis(2-methoxyethyl) ether, diethyl ether, toluene.

15. Process according to Claim 1 or any of Claims 2 to 14, conducted in the presence of moist air.

## Revendications

1. Procédé de fabrication de 2,2'-biarylène, selon lequel un mélange réactionnel contenant un premier aryle, un deuxième aryle et du dioxyde de sélénium est chauffé, le premier aryle étant mis en réaction avec le deuxième aryle pour former le 2,2'-biaryle correspondant, **caractérisé en ce que** le mélange réactionnel contient en outre du chlorure de molybdène (V).

2. Procédé selon la revendication 1, **caractérisé en ce que** le premier aryle et/ou le deuxième aryle sont un composé selon la formule générale I : 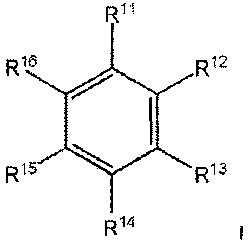 dans laquelle R¹¹, R¹², R¹³, R¹⁴, R¹³, R¹⁶ sont chacun choisis indépendamment les uns des autres parmi : -H, -alkyle en (C₁-C₁₂), -O-alkyle en (C₁-C₁₂), -aryle en (C₆-C₂₀), -O-aryle en (C₆-C₂₀), -Cl, -F, -I, -OC=O-alkyle en (C₁-C₁₂), -NH₂, -N [alkyle en (C₁-C₁₂)]₂ ;
deux radicaux voisins pouvant pouvant mais ne devant pas être reliés l'un à l'autre en un système condensé,
et les groupes alkyle et aryle mentionnés pouvant mais ne devant pas être substitués, et au moins un radical R¹¹, R¹², R¹³, R¹⁴, R¹⁵ ou R¹⁶ représentant -H.

3. Procédé selon la revendication 2, **caractérisé en ce que** R¹¹, R¹², R¹³, R¹⁴, R¹⁵, R¹⁶ chacun choisis indépendamment les uns des autres parmi : -H, -alkyle en (C₁-C₁₂), -O-alkyle en (C₁-C₁₂), -aryle en (C₆-C₂₀), -O-aryle en (C₆-C₂₀), -Cl, -F, -I, les groupes alkyle et aryle mentionnés pouvant mais ne devant pas être substitués, et au moins un radical R¹¹, R¹², R¹³, R¹⁴, R¹⁵ ou R¹⁶ représentant -H.

4. Procédé selon la revendication 2 ou 3, **caractérisé en ce que** le premier aryle et le deuxième aryle sont identiques.

5. Procédé selon la revendication 1, pour la fabrication de 2,2'-biphénols, **caractérisé en ce que** le premier aryle est un premier phénol, **en ce que** le deuxième aryle est un deuxième phénol et **en ce que** le premier phénol est mis en réaction avec le deuxième phénol pour former le 2,2'-biphénol correspondant.

6. Procédé selon la revendication 5, **caractérisé en ce que** le premier phénol et/ou le deuxième phénol sont un composé selon la formule générale II : 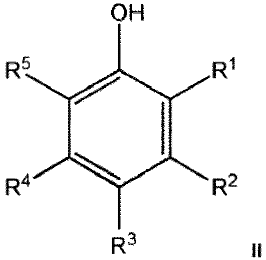 dans laquelle R¹, R², R³, R⁴, R⁵ sont chacun choisis indépendamment les uns des autres parmi : -H, -alkyle en (C₁-C₁₂), -O-alkyle en (C₁-C₁₂), -aryle en (C₆-C₂₀), -O-aryle en (C₆-C₂₀), -Cl, -F, -I, -OC=O-alkyle en (C₁-C₁₂), deux radicaux voisins pouvant mais ne devant pas être reliés l'un à l'autre en un système condensé,
les groupes alkyle et aryle mentionnés pouvant mais ne devant pas être substitués, et au moins R¹ ou R⁵ représentant -H.

7. Procédé selon la revendication 6, dans lequel R¹, R², R³, R⁴, R⁵ sont chacun choisis indépendamment les uns des autres parmi : -H, -alkyle en (C₁-C₁₂), -O-alkyle en (C₁-C₁₂), -aryle en (C₆-C₂₀), -0-aryle en (C₆-C₂₀), -Cl, - F, -I, les groupes alkyle et aryle mentionnés pouvant mais ne devant pas être substitués, et au moins R¹ ou R⁵ représentant -H.

8. Procédé selon la revendication 6 ou 7, **caractérisé en ce que** le premier phénol et le deuxième phénol sont identiques.

9. Procédé de fabrication de 2,2'-biarylène, **caractérisé en ce qu'**un éther 2,2'-sélénobiarylique est chauffé en présence de chlorure de molybdène (V) et transformé en un 2,2'-biaryle.

10. Procédé selon la revendication 11, dans lequel l'éther 2,2'-sélénobiarylique est un composé selon la formule générale III : 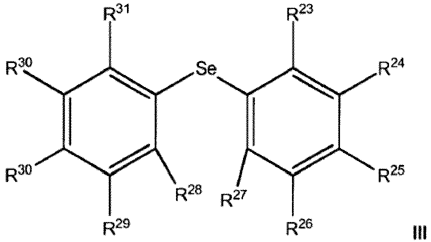 dans laquelle R²³, R²⁴, R²⁵, R²⁶, R²⁷, R²⁸, R²⁹, R³⁰, R³¹ sont chacun choisis indépendamment les uns des autres parmi : -H, -alkyle en (C₁-C₁₂), -O-alkyle en (C₁-C₁₂), -aryle en (C₆-C₂₀), -0-aryle en (C₆-C₂₀), -Cl, -F, -I, -OC=O-alkyle en (C₁-C₁₂), -NH₂, -N [alkyle en (C₁-C₁₂)]₂, OH ;
deux radicaux voisins pouvant mais ne devant pas être reliés l'un à l'autre en un système condensé,
et les groupes alkyle et aryle mentionnés pouvant mais ne devant pas être substitués.

11. Procédé selon la revendication 10, dans lequel R²³, R²⁴, R²⁵, R²⁶, R²⁷, R²⁸, R²⁹, R³⁰, R³¹ sont chacun choisis indépendamment les uns des autres parmi : -H, -alkyle en (C₁-C₁₂), -O-alkyle en (C₁-C₁₂), -aryle en (C₆-C₂₀), -O-aryle en (C₆-C₂₀), -Cl, -F, -I, OH.

12. Procédé selon la revendication 10 ou 11, dans lequel R²³, R²⁴, R²⁵, R²⁶, R²⁷, R²⁸, R²⁹, R³⁰, R³¹ sont chacun choisis indépendamment les uns des autres parmi : -H, -alkyle en (C₁-C₁₂), -O-alkyle en (C₁-C₁₂).

13. Procédé selon la revendication 1 ou selon l'une quelconque des revendications 2 à 13, réalisé en l'absence d'un solvant fluoré et/ou en l'absence d'un acide.

14. Procédé selon la revendication 1 ou selon l'une quelconque des revendications 2 à 13, réalisé en présence d'une base choisie parmi la pyridine, la quinoline, les bases aminées, le diméthylformamide et/ou en présence d'un solvant choisi parmi le tétrahydrofurane, l'éther diméthylique d'éthylène glycol, l'éther bis(2-méthoxyéthylique), l'éther diéthylique, le toluène.

15. Procédé selon la revendication 1 ou selon l'une quelconque des revendications 2 à 14, réalisé en présence d'air humide.
